Europäisches Patentamt

European Patent Office

Office européen des brevets

(19)

(11) Publication number: 0 269 870
A1

(12) # EUROPEAN PATENT APPLICATION

(21) Application number: 87115937.2

(22) Date of filing: 30.10.87

(51) Int. Cl.⁴ A61B 17/32 , A61F 9/00 ,
B06B 1/06 , B06B 3/02

(30) Priority: 07.11.86 US 929024

(43) Date of publication of application:
08.06.88 Bulletin 88/23

(84) Designated Contracting States:
AT BE CH DE ES FR GB GR IT LI LU NL SE

(71) Applicant: ALCON LABORATORIES INC
6201 South Freeway P.O. Box 1959
Fort Worth Texas 76101(US)

(72) Inventor: Lo, Ying-Ching,
820 Optimo Avenue,
Fremont, California 94539,(US)
Inventor: Hale, Reuben,
2238, Stuart Street,
Berkeley, California 94705(US)

(74) Representative: Dipl.-Ing. H. Marsch Dipl.-Ing.
K. Sparing Dipl.-Phys.Dr. W.H. Röhl
Patentanwälte
Rethelstrasse 123
D-4000 Düsseldorf(DE)

(54) Half wave, one node ultrasonic probe.

(57) There is disclosed herein a one node ultrasonic probe having a crystal transducer (24, 26) located as far as possible away from the single node (14). The probe is comprised of a rod (16, 18, 20) of metal having two different diameters (A, B) and a tapered section (18) between the two different diameters (A, B). The point of taper (18) marks the node (14) and is the point where mechanical connection to the probe is made so as to not dampen the vibrations of the probe at mechanical resonance. The excitation transducer is one or more piezoelectric crystal (24, 26) disks which are mechanically affixed in the larger diameter section (16) of the rod (16, 18, 20) at the end farthest from the node (14). A hollow needle threads into the end (22) of the rod (16, 18, 20) having the smaller diameter (C) and completes the one half wavelength of the probe. A fluid passageway passes through the rod (16, 18, 20) to couple the passageway in the needle to a nozzle on the other end of the rod (16, 18, 20). Tissue and fluid may be aspirated through this passageway.

FIG. 2

FIG. 1

# HALF WAVE, ONE NODE ULTRASONIC PROBE

## Background of the Invention

The invention pertains to the field of ultrasonic probes for use in ocular surgery.

In the field of ocular surgery, it is sometimes necessary to use ultrasonic probes to break up tissue formations in the eye such as cataracts. In the prior art, such probes have been constructed as metal rods having an ultrasonic transducer therein and a tapered section connecting a section of a larger diameter with a section of a smaller diameter the tip of which is used to contact the eye. The overall length of the rod from one end to the other end has, to date has been one wavelength of the driving frequency in length.

Typically, the driving frequency of such probes is around 40 kilohertz, and the probes have lengths of about 5 inches. Even if the probe is made out of a light metal such as titanium, a 5 inch metal rod weighs enough to make the small muscles of the hand tired after repeated manipulation of the probe over long periods of time in the cramped confines of the ocular surgery domain. This 5 inch length also results in a probe which is relatively awkward to move around. Further, if exotic metals such as titanium are used for the rod, the cost of materials for the rod may not be trivial.

The closes prior art of which the applicants are aware is the United Sonics probe. This probe may or may not be a one node probe since not enough is known about it to make that determination at this time. The driving frequency appears to be 27 kilohertz. If so, it is probably a one node probe. The problem with the United Sonics Probe is the placement of the crystals. In this probe, three crystals are used. The first set of crystals are the excitation transducers, and they are placed very near the single node of the probe on the large diameter side of the mechanical amplifier section. Another crystal is located near the antinode at the end of the probe opposite the needle end. This crystal appears to be a transducer used to detect the frequency of vibration of the probe or some other characteristic of the probe's vibration. The problem with placing the excitation crystals so close to the node is that it greatly increases the stress on the crystals. This increased stress greatly increases losses in the crystals and greatly increases heating of the probe from power dissipation in the crystals. That is, increased stress decreases the crystal Q factor and vastly increasing the amount of leakage current from one terminal to the other. The resulting changing temperature changes the elasticity of the probe material, and thus alters the mechanical resonant frequency of the probe. This can detune the probe thereby destroying the effectiveness of power transfer. Accordingly, a need has arisen for a short, lightweight ultrasonic probe which may be used in ocular surgery and which does not exhibit abnormally high stress or heat up from excessively high power dissipation in the crystals.

## Summary of the Invention

According to the teachings of the invention there is provided a half wave ultrasonic probe. The probe is approximately 2.5 inches in length, and has a single node and two antinodes. The probe is comprised of a cylinder of titanium or some other autoclavable material which can withstand the stress of mechanical resonance at approximately 40 kilohertz. The cylinder has an excitation section comprised of a cylinder of a first diameter which tapers down to a second smaller diameter through a mechanical amplifier and stress relief section. The purpose of the mechanical amplifier is to increase the amplitude of vibration of the tip of the probe in contact with the area being operated upon compared to the amplitude of the vibration of the probe at the end of the probe where the crystals are located. Preferably, the point at which the first section begins to taper down to the diameter of the second section is 1/4 wavelength. The portion of the rod having the second, smaller diameter extends out from the mechanical amplifier section and terminates in an end with a threaded hole therein. A third, cylindrical section of even smaller diameter threads into the hole in the end of the second diameter portion and completes the probe. It is this part which comes into contact with the eye during surgery. The overall length from the end of the third section which comes into contact with the eye to the farthest end of the first section is one half wavelength of the driving frequency. In the preferred embodiment, this driving frequency is approximately 40 kilohertz.

The probe has a pair of piezoelectric crystals which are mechanically affixed in the material of the first section of the probe. Preferably, these crystals are located at the opposite end of the first section from the point where the first section begins to taper. In the preferred embodiment, the single node, i.e., the point on the rod which does not vibrate when the rod is excited and vibrating at mechanical resonance, is coincident with the location on the first section where the diameter begins to taper to the diameter of the second section. The crystals are located in the first section. It has been found experimentally that if the crystals are located too close to the node, the stress on the crystal is high which causes the Q to decrease and leakage to increase. This causes the crystals to heat up and can melt the solder connections to the crystals. Conversely, if the crystals are located farther away from the node, the stress and leakage is lower so that less heating occurs, but the impedance of the crystals rises thereby requiring more voltage to drive them. The criteria for placement of the crystal should be set by the level of stress that the crystal can withstand. The two ends of the rod in the first and third sections are antinodes, i.e., points of maximum vibration excursion when the rod is resonating. Resonation of the rod is caused by driving the crystals with an electrical driving frequency which is equal to the mechanical resonance frequency of the rod. By placing the crystals on the rod as far away as possible from the node, the stress in the crystal during vibration is reduced, and the Q of the crystal remains high. This minimizes power dissipation losses in the crystal and keeps the probe cooler than would be the case if the crystals were located next to the node.

Brief Description of the Drawings

Figure 1 is a side view of the ultrosonic probe according to the teachings of the invention.

Figure 2 is a curve showing the comparative amounts of vibration excursion for points under the curve along the length of the probe during mechanical resonance.

Figure 3 is a cross sectional view of the endpiece which mates with the mating piece shown in Figure 4 to hold the crystal in place.

Figure 4 is a cross sectional view of the main body of the probe showing the internal aperture and the threaded apertures to mate with and hold the endpiece of Figure 3 and the hollow needle.

Detailed Description of the Preferred Embodiment

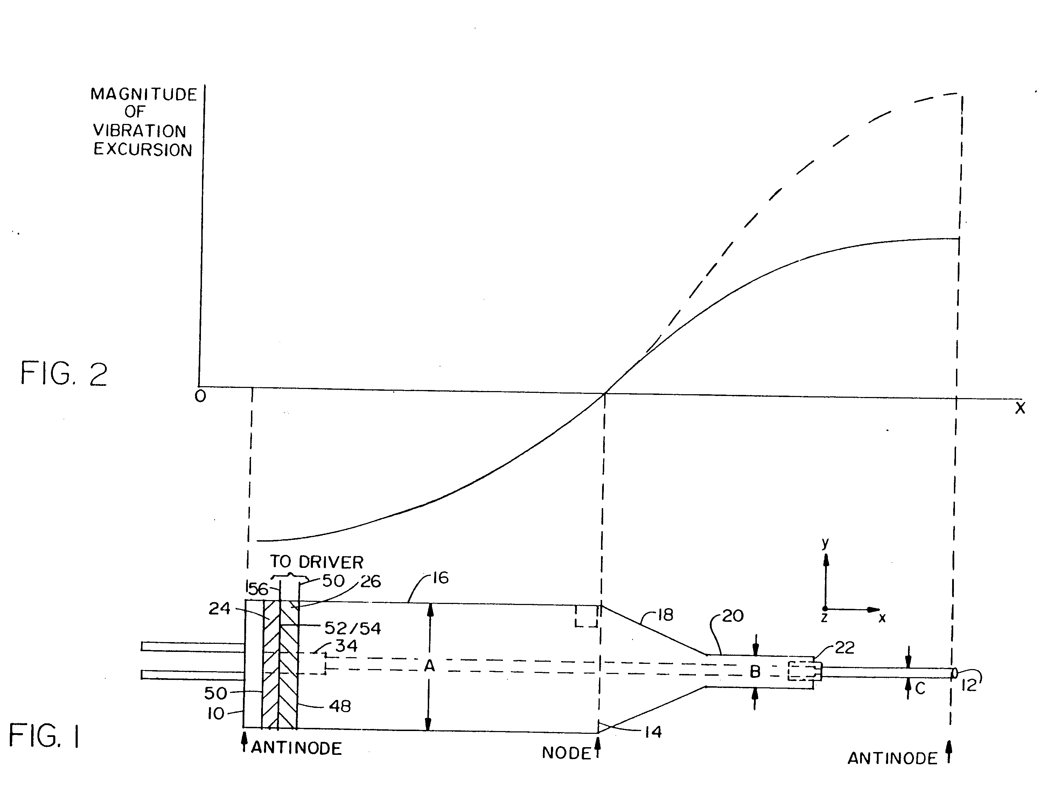

Referring to Figure 1, there is shown a side view of the ultrasonic probe of the invention. The probe has two antinodes at 10 and 12, and has a node at 14. A node is a place on the probe which does not vibrate when the probe in vibrating at mechanical resonance. An antinode is a place on the probe where the excursion of the vibration movement is maximum.

The probe is comprised of a first section 16 of diameter A terminating in a tapered section 18 which tapers down to the diameter of a second section 20 having a diameter B. The second section terminates in an end 22 having a threaded hole therein into which is threaded a third section of diameter C. The combined length of the second and third sections plus the tapered section is 1/4 wavelength.

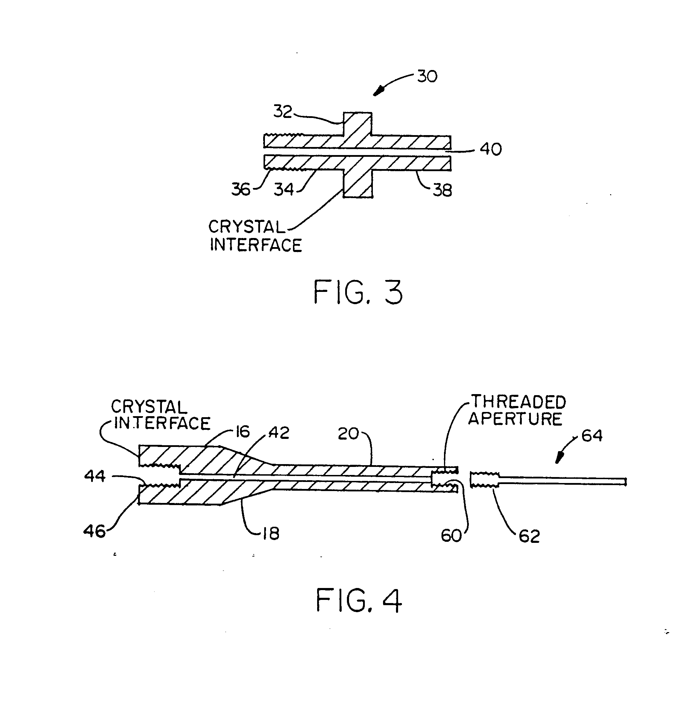

Two back to back piezoelectric crystals 24 and 26 are affixed in the material of the probe's first section to act as an excitation transducer. Typically, these crystals are bound together and to the material of the first section by a threaded stud 34 which passes through the two crystals and threads into threaded holes in the material of the first section of the rod. In the preferred embodiment, the threaded rod 28 is an extension of an end cap configured as shown in Figure 3 in cross section. The end cap 30 is made of conductive material with a shoulder 32 and a cylindrical projecting portion 34 having a threaded end 36. On the opposite side from the shoulder 32 is a nozzle over which surgical tubing may be placed to apply subatmospheric pressure to an aperture 40. The aperture 40 communicates with a similar aperture 42 in the mating section of the probe shown in Figure 4 which shows, in cross section, the section of the probe containing the first section 16, the tapered mechanical amplifier section 18 and the second section 20. The mechanical amplifier causes the cross sectional area of the probe section 16 to taper down to a much smaller cross sectional area on the probe section 20. The ratio of these two cross sectional area is the ratio by which the amplitude of the vibration of the tip 12 is increased over the amplitude of the vibration of the probe in the vicinity of the crystals.

The mating of the apertures is achieved when the threaded portion 36 of the cylindrical stud 34 is threaded into a threaded aperture 44 inside the first section 16. The aperture 42 passes completely through the mating section shown in Figure 4 and coupled to another threaded aperture 60 in the needle end of the probe. This aperture mates with the threaded end 62 of a hollow needle 64. This needle completes the probe when it is threaded into place, and has an aperture (not shown) which passes therethrough and is in fluid communication with the apertures 42 and 40 when the probe is assembled.

The crystals 24 and 26 are mechanically bound into position by the above noted mating process of the end piece 30 with the mating section shown in Figure 4. The crystals have holes formed therein which are large enough in diameter to clear the diameter of the stud 34. The stud is passed through these holes in the crystals prior to being threaded into the threaded aperture 44. As a result, the two crystals are squeezed between the crystal interface surface surface of the shoulder 32 and the crystal interface surface 46 of the mating section shown in Figure 4. Since in the preferred embodiment, the end piece and mating section are both made of metal such as titanium, the stud 34 serves to make an electrical connection between the surfaces 48 and 50 of the crystals. The crystals are oriented in such a way that surfaces 48 and 50 have the same polarity. Thus by placing an electrical contact 50 between the crystal surface 48 and the crystal interface surface, and another electrical contact 52 between the inner surfaces 52/54, separate electrical contacts may be made to the two crystals in parallel with each other with contact 50 acting as the positive or negative terminal, and contact 56 acting as the opposite polarity terminal.

When an alternating current driving signal is applied across contacts 50 and 56, the piezoelectric crystals vibrate at the frequency of the electrical driving frequency. This causes the material of the probe in Figure 1 to vibrate. The probe structure has a mechanical resonance frequency which is related to the mass of the probe and to the elasticity of the probe material. If the electrical frequency is close enough to the mechanical resonance frequency, mechanical resonance will exist in the probe and a standing wave of vibration will exist. This standing wave is illustrated in Figure 2 in two forms. The solid line represents the magnitude of the vibration excursion for all positions along the x axis of a probe with no mechanical amplifier section 18. The dashed line represents the vibration excursion for the probe with the mechanical amplifier section 18. The amount of mechanical excursion depends upon the ratios of the cross sectional areas of the first section 16 and the second section 20.

The crystals 24 and 26 are located as far away from the node as possible in the preferred embodiment. This minimizes the amount of stress on the crystal, and therefore minimizes the losses in the crystal. Experimental evidence indicates that when the crystals are located near the node 14, the stress on the crystal causes decreased Q and increased leakage between the electrical contacts 50 and 56. This leakage is represented by a greatly increased value for the resistance value in the equivalent circuit which represents the crystal and probe system. This vastly increased resistance corresponds to vastly increased power dissipation in the crystal and heating of the probe as a result. This heating of the probe alters the elasticity of the material of the probe and consequently changes the mechanical resonance frequency of the probe. This can detune the probe if the electrical driving frequency is not adjusted to match the shift in the mechanical resonance frequency.

Although the invention has been described in terms of the preferred embodiment illustrated herein, those skilled in the art will appreciate many modifications which do not depart from the spirit and scope of the invention as defined by the claims appended hereto. All such modifications are intended to be included within the scope of said claims.

## Claims

1. An ultrasonic probe for mechanically resonating at a frequency comprising:
a first cylinder having a first diameter;
a second cylinder having a second diameter;
a conical section joining said first cylinder to said second cylinder and matching the first and second diameters at the junction points; and
a piezoelectric transducer mechanically affixed to said first cylinder at the end opposite the juncture with said conical section,
wherein the overall length of said probe is one half the wavelength of said frequency and wherein the node of the standing wave at resonance is at the junction of said conical section with said first cylinder.

2. The probe of claim 1 wherein said first and second cylinders and said conical section are formed of titanium metal.

3. The probe of claim 1 wherein the first cylinder is comprised of an end piece having an annular shoulder having said first diameter surrounding a threaded stud and a mating piece comprised of a cylinder having said first diameter and having a threaded hole in one end surface of the cylinder having a diameter matching the threaded stud diameter for receiving said threaded stud and a pair of piezoelectric crystal disks having holes therein sufficiently large that said threaded stud may pass therethrough, said disks having said first diameter and placed between said annular shoulder and the end surface in said mating piece having the threaded hole therein whereby the crystal disks are mechanically bound to said first cylinder when said stud is engaged with the threaded hole in said mating piece.

4. An ultrasonic probe for resonating at a frequency comprising:
an end piece comprising a threaded stud protruding from an annular shoulder on one side, said annular shoulder having a nozzle projecting from the opposite surface from the surface from which said stud projects, said end piece having a fluid passageway passing axially through the threaded stud, said annular shoulder and said nozzle;
a mating piece comprising a cylinder of metal having a first diameter for a first segment of its length and then conically tapering down to a second diameter for a second segment of its length, and having a first threaded hole in the end surface having said first diameter with the diameter of the hole matching the diameter of the stud, and having said stud engaged therein, and having a second threaded hole in the end surface having said second diameter, and having a fluid passageway through the entire length of said mating piece opening onto said threaded holes so as to act as a continuation of the fluid passageway in said end piece;

a hollow needle having a third diameter and having a threaded end having a diameter matching the diameter of said second threaded hole, said hollow needle being threaded into said second hole such the fluid passageway in said hollow needle is in fluid communication with the fluid passageway in said mating piece; and

at least one piezoelectric crystal disk mechanically bound between said annular shoulder of said end piece and the surface of said mating piece having said first hole formed therein; and

means for making individual electrical contacts with the two terminals of opposite polarity of said crystal,

and wherein the overall length of said probe is one half wavelength and the node of the standing wave of mechanical resonance is at the point where said mating piece tapers from said first diameter to said second diameter and wherein the location of said crystal disk is as far removed as possible from the location of said node in the first diameter section of said mating piece.

5. An ultrasonic probe characterized by the fact that it is only one half wavelength long where the wavelength of measure is the wavelength of mechanical resonance.

6. An ultrasonic probe characterized by the fact that it is only one half wavelength long where the wavelength of measure is the wavelength of mechanical resonance and further characterized by the fact that the excitation transducer is located as far as possible away from the node location of maximum stress.

0 269 870

FIG. 2

MAGNITUDE OF VIBRATION EXCURSION

FIG. 1

ANTINODE

NODE

ANTINODE

TO DRIVER

32
30
36  34
40
38

CRYSTAL
INTERFACE

FIG. 3

CRYSTAL
INTERFACE   16  42   20   THREADED
APERTURE   64

44   18
46   60   62

FIG. 4

## DOCUMENTS CONSIDERED TO BE RELEVANT

EP 87115937.2

| Category | Citation of document with indication. where appropriate, of relevant passages | Relevant to claim | CLASSIFICATION OF THE APPLICATION (Int. Cl 4) |
|---|---|---|---|
| X | US - A - 4 173 725 (K. ASAI et al.) | 5 | A 61 B 17/32 |
| A | * Fig. 1A,1B,8ACi), 8ACii); column 1, line 60 - column 2, line 55; column 17, lines 19-48; claim 10 * | 1,4,6 | A 61 F 9/00 |
| | | | B 06 B 1/06 |
| | | | B 06 B 3/02 |
| | -- | | |
| A | US - A - 4 169 984 (T. PARISI) | 1,4 | |
| | * Fig. 2,3,8; column 4, lines 58-68; column 5, lines 11-42, 50-52; column 5, line 66 - column 6, line 47; column 7, lines 22-29 * | | |
| | -- | | |
| A | GB - A - 2 032 221 (KEELER INSTR.) | 1 | |
| | * Fig.; page 2, lines 87-119 * | | |
| | -- | | **TECHNICAL FIELDS SEARCHED (Int. Cl 4)** |
| A | US - A - 4 504 264 (CH.D. KELMAN) | 1,4 | |
| | * Fig. 1; column 2, lines 33-36, 53-56 * | | A 61 B 17/00 |
| | -- | | A 61 F 9/00 |
| P,A | GB - A - 2 176 110 (NIPPON INFRA-RED IND.) (17-12-1986) | 1 | B 06 B 1/00 |
| | | | B 06 B 3/00 |
| | * Fig. 1,3; page 2, lines 17-29; page 3, lines 43-53 * | | |
| | & FR-A -2 582 506 (05-12-1986) & JP-A2-61 279 239 (10-12-1986) & DE-A1-3 527 586 (11-12-1986) | | |
| | ---- | | |

The present search report has been drawn up for all claims

| Place of search | Date of completion of the search | Examiner |
|---|---|---|
| VIENNA | 16-03-1988 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82